# EUROPEAN PATENT APPLICATION

(11) **EP 3 799 845 A1**
(43) Date of publication of application: **07.04.2021**
(21) Application number: 20382795.1
(22) Date of filing: 08.09.2020
(51) Int. Cl.: A61F 13/00, A61F 13/02, A61F 13/82, A61F 13/84

(54) **PERSONAL HYGIENE ITEM**

(30) Priority: 30.09.2019 ES 201931574 U
(71) Applicant: Sanchez Casadevall, Enrique, 17600 Figueres (ES)
(72) Inventor: Sanchez Casadevall, Enrique, 17600 Figueres (ES)
(74) Representative: Munoz Garcia, Antonio

(57) **Abstract**

The present invention relates to a personal hygiene item, applicable for being attached to the anus after defecation and for serving as a means for isolating clothing from possible fecal remains that are retained in said area after a bowel movement, said personal hygiene item being configured from a disposable piece of paper (2) having a variable shape and size, incorporating disinfectant and absorbent product (3) and provided with adhesive (4) so that it can stick to the skin in the area of the anus, covering it completely. The disposable piece of paper (2) has a central area (2a) impregnated with the disinfectant product and absorbent elements (3) which is surrounded by a perimetral area (2b) provided with an adhesive (4) on one of the faces thereof that will be placed against the body. The disinfectant product is of the type that does not irritate the skin and the adhesive (4) is non-allergic and removable.

## Description

### OBJECT OF THE INVENTION

As expressed in the title of the present specification, the invention relates to a personal hygiene item which provides the intended function thereof with the advantages and features that are described in detail below and constitute a novelty.

More specifically, the object of the invention focuses on a new hygiene item, specifically designed for being attached to the anus and for isolating clothing from possible fecal remains that are retained in said area after defecation, for which said personal hygiene item is configured from a small disposable piece of paper containing disinfectant and absorbent product, and is provided with an adhesive so that it can stick to the skin.

### FIELD OF APPLICATION OF THE INVENTION

The field of application of the present invention is comprised within the sector of the industry dedicated to the manufacturing of hygiene products, focusing particularly on the field of disposable personal hygiene and intimate hygiene products.

### BACKGROUND OF THE INVENTION

As is known, although it seems like the anus is clean when using toilet paper alone after defecation, given the particular anatomical structure of the anus which furthermore often becomes deformed particularly with age and/or due to some pathologies, there are always fecal remains or traces retained between the folds of the sphincter if it is not washed with soap and water, causing clothing that is in contact with this area to become dirty, and accordingly to retain bacteria which, in addition to an unpleasant odor, can entail a health risk.

To that end, and given that it is not possible to perform said washing with soap and water in most cases, the objective of the present invention is to provide the market with a new type of personal hygiene item which solves said problems in a simple and practical manner by providing very easy to use isolating and disinfecting means.

Furthermore, and as an additional effect, the development of this item may contribute to preventing or reducing the increased use of wet wipes which, as is known, are posing a serious environmental and sanitary sewer cleaning problem, because they are manufactured from materials that cannot be readily destroyed and many users throw them into the toilet after cleaning themselves with said wipes, causing the sewers of many cities to clog up significantly.

Although these wipes, which are usually impregnated with a soapy product, entail a more thorough cleaning of the anus as a result of said products, they do not ensure a complete elimination of the fecal traces retained between the folds of the sphincter either, so it would likewise be desirable to use an isolating element which is placed between the skin and the clothing, like the item herein proposed, so it would be easier and more cost-effective to first use only conventional toilet paper and then this isolating item, preventing the use of wet wipes.

Moreover, and as a reference to the current state of the art, it should be pointed out that although a variety of personal hygiene items are available on the market, the applicant at least is unaware of the existence of any item, applicable for being attached to the skin and for serving as an isolating element between the area of the anus and the clothing, having technical, structural, and constitutive features that are identical or similar to those herein claimed.

### DESCRIPTION OF THE INVENTION

The personal hygiene item proposed by the invention allows achieving the objectives indicated above in a satisfactory manner, with the characterizing details making it possible and distinguishing it being suitably described in the final claims accompanying the present description.

Specifically, as indicated above, the invention proposes a hygiene item designed for the purpose of being fixed to the anus of the user after defecation and for serving as a means for isolating clothing from possible fecal remains that are retained in the skin of said area after a bowel movement, providing disinfection and absorbance.

To that end, and more specifically, said item is configured from a small disposable piece of paper containing a disinfectant and absorbent and is furthermore provided with an adhesive so that it can stick to the skin.

In the preferred embodiment, the disposable piece of paper has a central area impregnated with a disinfectant product, preferably of the type that does not irritate the skin, and provided with absorbent elements, said central area being surrounded by a perimetral area provided, on one of the faces thereof that will be placed against the body, with an adhesive product suitable for being used on the skin, preferably a non-allergic and removable adhesive, for example a water glue-based adhesive.

Optionally, the piece of paper incorporates a thin layer of waterproof material on the face opposite the face which is adhered to the skin. Furthermore, a protective film which is removed before use will logically be provided on the face incorporating the adhesive.

Finally, it should be pointed out that the piece of paper forming the described hygiene item may have different shapes and dimensions, preferably those that better adapt to the anatomy of the area to achieve suitable fixing between the buttocks, without causing the user any discomfort when walking.

### DESCRIPTION OF THE DRAWINGS

To complement the description that is being made and for the purpose of helping to better understand the features of the invention, a set of drawings is attached to the present specification as an integral part thereof in which the following is depicted in an illustrative and non-limiting manner:
Figure 1 shows a schematic perspective view of an example of the personal hygiene item object of the invention, where the parts and elements it comprises, as well as the configuration and arrangement thereof, can be seen.
Figure 2 shows a schematic view of the way in which the personal hygiene item of the invention is attached to the body for use.

### PREFERRED EMBODIMENT OF THE INVENTION

In view of the mentioned figures and according to the reference numbers used, a non-limiting embodiment of the personal hygiene item of the invention, which comprises everything indicated and described in detail below, can be seen therein.

Therefore, as seen in said figures, the item (1) at hand, applicable for being attached to the anus of a user after defecation and for serving as a means for isolating clothing from possible fecal remains that are retained in said area after a bowel movement, is configured from a disposable piece of paper (2) having a variable shape and size, incorporating disinfectant and absorbent product (3) and furthermore provided with an adhesive (4) so that it can stick to the skin in the area of the anus, covering it completely.

In the preferred embodiment, the disposable piece of paper (2) has a central area (2a) impregnated with the disinfectant product and absorbent elements (3) which is surrounded by a perimetral area (2b) provided with an adhesive (4) on one of the faces thereof that will be placed against the body.

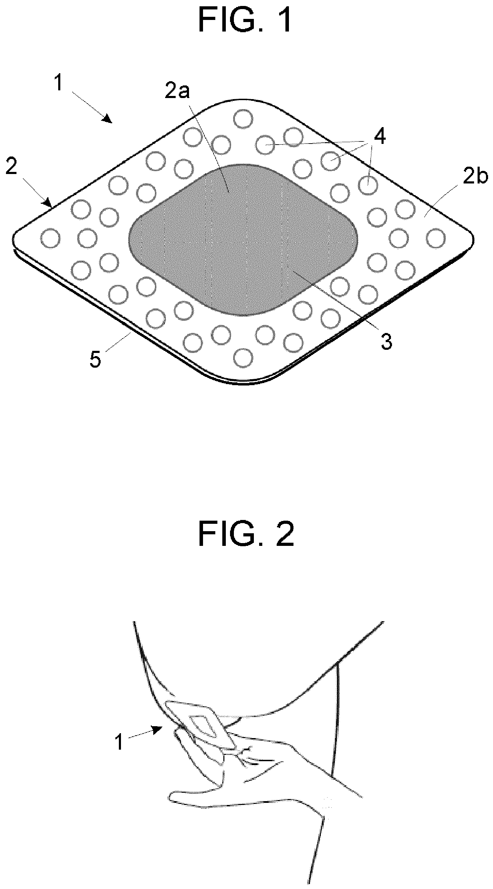

In any case, the disinfectant product is preferably of the type that does not irritate the skin, and the adhesive (4) is non-allergic and removable, for example a water glue-based adhesive, where it can be spread out in small dots, as shown in the example of Figure 1.

Optionally, the piece of paper (2) incorporates a thin layer of waterproof material (5) on the face opposite the face incorporating the adhesive (4) with which it is adhered to the skin.

Having sufficiently described the nature of the present invention, as well as the manner of putting it into practice, it is not considered necessary to expand on this explanation so that one skilled in the art will understand its scope and the advantages derived from it, hereby stating that within its essential nature, the invention may be carried out to practice in other embodiments which differ in detail from that indicated by way of example and which will likewise attain the protection that is sought provided that its fundamental principle is not altered, changed, or modified.

## Claims

1. A personal hygiene item, applicable for being attached to the anus of a user after defecation and for serving as a means for isolating clothing from possible fecal remains that are retained in said area after a bowel movement, **characterized by** being configured from a disposable piece of paper (2) having a variable shape and size, incorporating disinfectant and absorbent product (3) and provided with adhesive (4) so that it can stick to the skin in the area of the anus, covering it completely.

2. The personal hygiene item according to claim 1, **characterized in that** the disposable piece of paper (2) has a central area (2a) impregnated with the disinfectant product and absorbent elements (3) which is surrounded by a perimetral area (2b) provided with an adhesive (4) on one of the faces thereof that will be placed against the body.

3. The personal hygiene item according to claim 1 or 2, **characterized in that** the disinfectant product is of the type that does not irritate the skin.

4. The personal hygiene item according to any of claims 1 to 3, **characterized in that** the adhesive (4) is non-allergic and removable.

5. The personal hygiene item according to any of claims 1 to 4, **characterized in that** the piece of paper (2) incorporates a thin layer of waterproof material (5) on the face opposite the face incorporating the adhesive (4) with which it is adhered to the skin.
